# DEMANDE DE BREVET EUROPEEN

(11) **EP 2 277 502 A2**
(43) Date de publication de la demande: **26.01.2011**
(21) Numéro de dépôt: 10186173.0
(22) Date de dépôt: 29.03.2006
(51) Int. Cl.: A61K 8/97, A61Q 19/04, A61Q 1/02

(54) **Utilisation d'extraits végétaux pour teinter la peau en fonction de son phototype**

(30) Priorité: 31.03.2005 FR 0503171; 07.07.2005 US 177698
(62) Demande divisionnaire de: 06290493.3
(71) Demandeur: BASF Beauty Care Solutions France SAS, 69007 Lyon (FR)
(72) Inventeur: Abdul-Malak, Nabil, 69300, Caluire (FR); Perrier, Eric, 38138, Les Cotes D'Arey (FR)
(74) Mandataire: Portal, Frédéric

(57) **Abrégé**

L'invention concerne l'utilisation d'un extrait végétal de la famille des composées ou astéracées, de préférence un extrait de chicorée sauvage (Cichorium intybus L.), pour teinter, en particulier pour colorer, un tissu cutané d'un sujet, de préférence pour augmenter l'aspect bronzé de ce tissu.

L'invention concerne également un procédé de criblage d'un végétal pour la fabrication d'une composition cosmétique pour colorer, en particulier pour teinter, au moins une partie d'un tissu cutané d'un sujet.

## Description

La présente invention concerne un composé pour teinter la peau, notamment à base d'un extrait végétal et/ou d'un mélange d'extraits végétaux. La présente invention concerne en particulier la préparation d'un composé modulée en fonction du phototype de peau claire ou mate à colorer.

### ETAT DE L'ART

La DHA (dihydroxyacétone) est aujourd'hui une des seules molécules utilisée dans le domaine de la cosmétique pour sa capacité à teinter la peau. Elle n'agit pas sur la mélanogénèse mais colore la peau, par réaction avec les fonctions amines portées par les acides aminés des protéines et peptides de la peau. D'autres molécules sont parfois utilisées en synergie avec la DHA : l'érythrulose qui semble augmenter l'activité de cette réaction, ou les AHA (Alpha HydroxyAcides) qui semblent permettre une meilleure uniformité de la couleur obtenue. L'ajout d'acides aminés dans les formulations pour permettre un meilleur contrôle de la teinte obtenue sur la peau, a été imaginé par de nombreux formulateurs, sans réel succès jusqu ici, la DHA réagissant avec les acides aminés dans la formulation.

L'inconvénient principal de la DHA est l'apparition de couleur et d'odeur dans les formules qui en utilisent.

Afin d'éviter les inconvénients de la DHA, certaines formulations teintantes ont été imaginées et proposées sur le marché cosmétique. Ces solutions teintantes, qui ne contiennent pas de DHA, s'appliquent à toutes les peaux sans prendre en considération les différents phototypes de la peau ni la comparaison avec le bronzage naturel de celle-ci. La conséquence est l'obtention de couleurs parfois très éloignées d'un bronzage naturel (couleur verdâtre par exemple).

Il n'existe donc pas à ce jour de composition cosmétique essentiellement sans DHA, adaptée au problème consistant en l'obtention d'une modification de la couleur de la peau pour reproduire un bronzage naturel, ce bronzage étant différent en fonction du phototype de la personne.

### BUTS DE L'INVENTION

L'invention a pour but principal de résoudre le problème technique consistant en la fourniture d'une composition teintante, en particulier colorante, à base d'au moins un extrait d'un végétal, permettant d'obtenir après application sur la surface cutanée, d'obtenir un effet « bonne mine » et en particulier une couleur de peau reproduisant la couleur naturelle du bronzage.

L'invention a pour but en particulier de résoudre le problème technique consistant en la fourniture de plusieurs solutions teintantes, en particulier colorantes, à base d'extraits végétaux, permettant d'obtenir après application sur la surface cutanée, une couleur de peau reproduisant la couleur naturelle du bronzage, chaque composition teintante étant adaptée à un phototype particulier.

La présente invention a pour but de fournir une composition cosmétique permettant d'obtenir un effet « bonne mine ».

La présente invention a également pour but de fournir une composition cosmétique permettant de modifier la teinte de la peau, en particulier en colorant le tissu cutané pour obtenir une coloration proche du bronzage naturel.

L'ensemble de ces problèmes techniques est résolu pour la première fois d'une manière satisfaisante, peu coûteuse, utilisable à l'échelle industrielle, notamment en cosmétique.

### DESCRIPTION DE L'INVENTION

Les produits issus de cette invention présentent un effet teintant, en particulier colorant, lors de l'application sur la peau d'une composition les contenant.

Les produits de l'invention s'adsorbent à la surface de la peau et donnent une couleur très proche du bronzage naturel lorsqu'ils sont utilisés à une concentration efficace.

Lorsque les produits de l'invention sont utilisés à une concentration inférieure à la concentration efficace, les produits de l'invention permettent d'obtenir une teinte suffisante pour avoir un effet « bonne mine ».

Ainsi les inventeurs entendent par «teinter» l'obtention d'une variation de l'aspect du tissu cutané reflétant l'aspect « bonne mine » ainsi que l'obtention d'une variation de l'aspect du tissu cutané reproduisant l'aspect bronzé.

Avantageusement, l'aspect « bonne mine » est apprécié par un panel d'experts.

La concentration efficace peut être aisément appréciée par l'homme de l'art. Elle permet d'obtenir une variation de la coloration de la peau proche de celle du bronzage naturel. Pour apprécier avec plus de précisions la variation de la coloration de la peau, l'homme de l'art peut se référer à la variation de l'angle de typologie individuelle ou ITA, ou à la variation du paramètre b, comme illustré ci-après.

Les produits de la présente invention permettent avantageusement en fonction du phototype, de reproduire un bronzage proche de celui obtenu naturellement. En particulier, les produits de l'invention donnent une couleur très proche du bronzage naturel et peuvent être adaptés aux phototypes I et II (peaux claires) et III et IV (peaux mates).

Selon un premier aspect, l'invention concerne un procédé de criblage d'un extrait végétal pour la fabrication d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage ;
b) l'application d'un mélange d'un extrait végétal ou d'un mélange d'extraits végétaux sur au moins une partie d'un tissu cutané ;
c) la sélection de l'extrait végétal ou du mélange d'extraits végétaux permettant de reproduire en b), la variation de l'intensité de couleur obtenue à l'étape a).

Selon un mode de réalisation, l'invention met en oeuvre une phase de criblage effectuée sur des modèles cutanés, comprenant au moins un épiderme.

Avantageusement, les modèles cutanés sont des biopsies ou des modèles de peau connue de l'homme de l'art.

Selon un autre mode de réalisation, l'invention met en oeuvre une phase de criblage effectuée sur des êtres humains volontaires.

De préférence, l'invention met en oeuvre les deux étapes avec de préférence une première phase de criblage effectué sur des modèles cutanés et une seconde phase de criblage effectué sur des êtres humains volontaires.

De préférence, le procédé de criblage d'un extrait végétal pour la fabrication d'une composition cosmétique pour teinter, en particulier pour colorer, au moins une partie d'un tissu cutané d'un sujet comprend :
a) la détermination de la variation de l'intensité de couleur d'un tissu cutané avant et après bronzage;
b) l'application d'un mélange d'un extrait végétal de la famille des composées ou astéracées, de préférence un extrait de chicorée sauvage, et d'un autre extrait végétal sur au moins une partie d'un tissu cutané ;
c) la sélection de cet autre extrait végétal lorsque la variation de l'intensité de couleur du tissu cutané avant et après application à l'étape b) reproduit la variation de l'intensité de couleur obtenue à l'étape a).

Avantageusement, invention consiste dans un premier temps, à mesurer les couleurs de tissus cutanés de phototypes différents, avant puis après leur bronzage dans des conditions normales, puis à en déduire les évolutions de couleur obtenues selon les critères de mesure de la couleur bien connus de l'homme de l'art (mesure des paramètres L (luminosité), a et b (décomposition de la couleur)).

L'invention consiste alors à trouver des végétaux et/ou extraits de végétaux (seuls ou en mélanges) capables de reproduire les variations de couleur ainsi observées, lorsqu'ils sont utilisés à une concentration efficace.

Lorsque les produits de l'invention sont utilisés à une concentration inférieure à la concentration efficace, les produits de l'invention permettent d'obtenir une teinte suffisante pour avoir un effet « bonne mine ».

Avantageusement, suite à la sélection d'un végétal et/ou d'un extrait végétal, il est réalisé la préparation d'une composition à l'usage topique, en particulier d'une composition cosmétique.

Selon un second aspect, invention concerne également un procédé de préparation d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage;
b) l'application d'un mélange d'un extrait végétal ou d'un mélange d'extraits végétaux sur au moins une partie d'un tissu cutané ;
c) la sélection de l'extrait végétal ou du mélange d'extraits végétaux lorsque la variation de l'intensité de couleur du tissu cutané avant et après application à l'étape b) reproduit la variation de l'intensité de couleur obtenue à l'étape a).
d) la formulation de cet extrait végétal ou de ce mélange d'extraits végétaux pour obtenir une composition cosmétique permettant de teinter, en particulier pour colorer au moins une partie d'un tissu cutané d'un sujet.

De préférence, le procédé de préparation d'une composition cosmétique pour teinter, en particulier pour colorer, au moins une partie d'un tissu cutané d'un sujet comprend :
a) la détermination de la variation de l'intensité de couleur d'un tissu cutané avant et après bronzage;
b) l'application d'un mélange d'un extrait végétal de la famille des composées ou astéracées, de préférence un extrait de chicorée sauvage, et d'un autre extrait végétal sur au moins une partie d'un tissu cutané ;
c) la sélection de cet autre extrait végétal lorsque la variation de l'intensité de couleur du tissu cutané avant et après application à l'étape b) reproduit la variation de l'intensité de couleur obtenue à l'étape a) ;
d) la formulation du mélange de l'extrait de ce végétal et de l'extrait végétal de la famille des composées ou astéracées, de préférence de l'extrait de la chicorée sauvage, pour obtenir une composition cosmétique pour teinter, en particulier pour colorer au moins une partie d'un tissu cutané d'un sujet.

Avantageusement, les tissus cutanés utilisés dans l'étape b) sont tels que définis précédemment.

Le protocole cité ci-dessus a permis la reconnaissance de composés adaptés à la présente invention.

Il a été découvert de manière inattendue qu'un extrait végétal de la famille des composées ou astéracées, de préférence un extrait de chicorée sauvage (Cichorium intybus L.) permet de teinter, en particulier colorer, un tissu cutané.

Ainsi, selon un troisième aspect, l'invention concerne l'utilisation d'un extrait végétal de la famille des composées ou astéracées, de préférence un extrait de chicorée sauvage ou commune (Cichorium intybus L.), pour teinter un tissu cutané d'un sujet, et en particulier pour colorer ce tissu cutané de préférence pour augmenter son aspect bronzé ou doré (couleur jaune).

Avantageusement, l'extrait végétal de la famille des composées ou astéracées, de préférence un extrait de chicorée sauvage, est utilisé en combinaison avec un autre extrait végétal, en particulier pour obtenir un effet supplémentaire de teinte, en particulier de coloration du tissu cutané. L'extrait végétal de la famille des composées permet d'obtenir une variation de teinte tandis que l'autre extrait végétal permet d'affiner la variation de la teinte pour en particulier s'adapter aux différents phototypes.

Selon un quatrième aspect, l'invention concerne l'utilisation d'un extrait végétal de la famille des composées ou astéracées, de préférence un extrait de chicorée sauvage, pour teinter, en particulier pour colorer, un tissu cutané de phototype I et/ou II d'un sujet, de préférence pour augmenter l'aspect bronzé ou doré (couleur jaune) de ce tissu.

Avantageusement, l'extrait végétal de la famille des composées ou astéracées, de préférence un extrait de chicorée sauvage, est utilisé en combinaison avec un extrait de la famille des Oléacées, comme par exemple un extrait de Muirapuama ou Marapuama (Ptychoetalum olacoides).

Selon un cinquième aspect, l'invention concerne l'utilisation d'un extrait végétal de la famille des composées ou astéracées, de préférence un extrait de chicorée sauvage, pour teinter, en particulier pour colorer, un tissu cutané de phototype III et/ou IV d'un sujet, de préférence pour augmenter l'aspect bronzé de ce tissu.

Avantageusement, l'extrait végétal de la famille des composées ou astéracées, de préférence un extrait de chicorée sauvage, est en combinaison avec un extrait de la famille des asclépiadacées, comme par exemple un extrait de Gymnema sylvestre.

La présente invention couvre avantageusement selon le troisième, quatrième ou cinquième aspect, l'utilisation desdits extraits pour reproduire le bronzage naturel de la peau d'un être humain.

En particulier il est tout à fait avantageux de calculer la valeur de l'angle de typologie individuelle (ITA) qui répond à la formule simplifiée :
**ITA= arc tang [(L*-50)/b*],** où L* et b* correspondent respectivement à la valeur moyenne de la luminosité (du blanc +L au noir -L) et de l'aspect «jaune-bleu» (du jaune +b au bleu -b). Une diminution du paramètre **ITA** correspond à un assombrissement de la peau et à une augmentation des pigments mélaniques.

La valeur du paramètre ITA observée après un bronzage naturel est égale à environ 70% de la valeur ITA initiale. Les produits de l'invention permettent d'obtenir des variations similaires (ITA après application compris par exemple entre 50% et 90% de la valeur initiale).

Selon un sixième aspect, l'invention concerne également l'utilisation d'un extrait végétal de la famille des composées ou astéracées, de préférence un extrait de Chicorée sauvage ou commune (Cichorium intybus L.), mélangé à un autre extrait végétal pour obtenir une variation de teinte, en particulier pour reproduire les variations de couleur observées lors du bronzage naturel.

Avantageusement, l'invention concerne d'un extrait végétal de la famille des composées ou astéracées, de préférence un extrait de chicorée sauvage, en combinaison avec un extrait végétal choisi parmi le groupe consistant en :
Menthe piperta; Malva silvestris; Cynara scolymus; Thea sinensis; Juglans regia ; Lawsonia inermis; Castanea vulgaris; Asarum europaeum; Leonurus cardiaca; Ballota foetida ; Ocimum basilicum ; Stachys officinalis; Brunella vulgaris ; Calamintha officinalis ; Thymus vulgaris ; Rosmarinus officinalis ; Humulus lupulus ; Vaccinium myrtillus ; Arctotaphylos uva-ursi ; Calluna vulgaris ; Artemisia abisinthium ; Artemisia vulgaris, Artemisia abrotonum ; Artemisia glacialis ; Artemisia mutellina ; Artemisia spicata ; Chamaemelum nobile ; Fraxinus excelsior ; Syringa vulgaris ; Jasminium grandiflorum ; Lythrum salicaria ; Althaea officinalis ; Hysopus officinalis ; Origanum majorana ; Salvia officinalis ; Melissa officinalis ; Melittis melissophyllum ; Lavandula officinalis, Quercus robur ; Fagus silvatica ; Nepta cataria ; Origanum dictamus ; Thymus serpyllum, pour reproduire un bronzage naturel sur au moins une partie du tissu cutané d'un sujet.

Selon un septième aspect, l'invention concerne une composition, en particulier d'une composition cosmétique comprenant un extrait végétal de la famille des composées ou astéracées, de préférence un extrait de chicorée sauvage, et un extrait d'un végétal choisi parmi le groupe défini précédemment.

Selon un huitième aspect, l'invention concerne un procédé de préparation d'une telle composition.

Avantageusement, le procédé comprend la préparation des mélanges d'extraits végétaux qui comprend les étapes consécutives suivantes consistant à fabriquer dans un premier temps un extrait végétal de la famille des composées ou astéracées, de préférence un extrait de chicorée sauvage, et un extrait d'un autre végétal, en particulier choisi parmi ceux énumérés précédemment, de préférence choisie parmi un extrait de gymnema, un extrait de muirapuama, et leur combinaisons, puis de les mélanger dans des proportions adaptées au phototype à traiter, comme par exemple environ 65 à 85%, de préférence environ 75%, d'extraits végétal de la famille des composées ou astéracées, de préférence un extrait de chicorée, et environ 15 à 35%, de préférence environ 25%, d'autres extraits de végétaux efficaces pour modifier la coloration de la peau notamment pour reproduire un bronzage naturel.

Avantageusement, le second extrait de végétal sera choisi parmi le groupe de végétaux consistants en :
Menthe piperta ; Malva silvestris ; Cynara scolymus ;Thea sinensis ;Juglans regia ; Lawsonia inermis ; Castanea vulgaris ; Asarum europaeum ; Leonurus cardiaca; Ballota foetida ; Ocimum basilicum ; Stachys officinalis ; Brunella vulgaris ; Calamintha officinalis ; Thymus vulgaris ; Rosmarinus officinalis ; Humulus lupulus ; Vaccinium myrtillus ; Arctotaphylos uva-ursi ; Calluna vulgaris ; Artemisia abisinthium ; Artemisia vulgaris, Artemisia abrotonum ; Artemisia glacialis ; Artemisia mutellina ; Artemisia spicata ; Chamaemelum nobile ; Fraxinus excelsior ; Syringa vulgaris ; Jasminium grandiflorum ; Lythrum salicaria ; Althaea officinalis ; Hysopus officinalis ; Origanum majorana ; Salvia officinalis ; Melissa officinalis ; Melittis melissophyllum ; Lavandula officinalis, Quercus robur ; Fagus silvatica ; Nepta cataria ; Origanum dictamus ; Thymus serpyllum.

Les produits selon la présente invention sont préparés sous forme de compositions topiques, notamment sous forme de compositions cosmétiques ou dermo-pharmaceutiques. De ce fait, pour ces compositions, l'excipient contient par exemple au moins un composé choisi parmi le groupe consistant en les conservateurs, les émollients, les émulsifiants, les tensioactifs, les hydratants, les épaississants, les conditionneurs, les agents matifiants, les stabilisants, les antioxydants, les agents de texture, les agents de brillance, les agents filmogènes, les solubilisants, les pigments, les colorants, les parfums et les filtres solaires. Ces excipients sont de préférence choisis parmi le groupe consistant en les acides aminés et leurs dérivés, les polyglycérols, les esters, les polymères et dérivés de cellulose, les dérivés de Lanoline, les phospholipides, les lactoferrines, les lactoperoxidases, les stabilisants à base de sucrose, les vitamines E et ses dérivés, les cires naturelles et synthétiques, les huiles végétales, les triglycérides, les insaponifiables, les phytosterols, les esters végétaux, les silicones et ses dérivés, les hydrolysats de protéines, l'huile de Jojoba et ses dérivés, les esters lipo/hydrosolubles, les betaines, les aminoxides, les extraits de plantes les esters de Saccharose, les dioxydes de Titane, les glycines, et les parabens, et encore de préférence parmi le groupe consistant en le butylène glycol, le stéareth-2, le stéareth-21, le glycol-15 stéaryl éther, le cétéaryl alcool, le phénoxyéthanol, le méthylparaben, l'éthylparaben, le propylparaben, le butylparaben, le butylène glycol, les tocopherols naturels, la glycérine, le sodium dihydroxycétyle, l'isopropyl hydroxycétyl éther, le glycol stéarate, le triisononaoine, l'octyl cocoate, le polyacrylamide, l'isoparaffine, le laureth-7, un carbomer, le propylène glycol, le glycérol, le bisabolol, une diméthicone, l'hydroxyde de sodium, le PEG 30-dipolyhydroxystérate, les caprique/caprylique triglycérides, le cétéaryl octanoate, le dibutyl adipate, l'huile de pépin de raisin, l'huile de jojoba, le sulfate de magnésium, l'EDTA, une cyclométhicone, la gomme de xanthane, l'acide citrique, le lauryl sulfate de sodium, les cires et les huiles minérales, l'isostéaryl isostéarate, le dipélargonate de propylène glycol, l'isostéarate de propylène glycol, le PEG 8 Beewax, les glycérides d'huile de coeur de palme hydrogénée, les glycérides d'huile de palme hydrogénée, l'huile de lanoline, l'huile de sésame, le cétyl lactate, le lanoline alcool, l'huile de ricin, le dioxyde de titane, le lactose, le saccharose, le polyéthylène basse densité, une solution isotonique salée.

Avantageusement, les compositions précitées sont formulées sous une forme choisie parmi le groupe consistant en une solution, aqueuse ou huileuse, une crème ou un gel aqueux ou un gel huileux, notamment en pot ou en tube, notamment un gel douche, un shampoing ; un lait ; une émulsion, une microémulsion ou une nanoémulsion, notamment huile-dans-eau ou eau-dans-huile ou multiple ou siliconée ; une lotion, notamment en flacon de verre, de plastique ou en flacon doseur ou en aérosol ; une ampoule ; un savon liquide ; un pain dermatologique ; une pommade ; une mousse ; un produit anhydre, de préférence liquide, pâteux ou solide, par exemple sous forme de bâtonnet, notamment sous forme de rouge à lèvre.

D'autres buts, caractéristiques et avantages de l'invention apparaîtront clairement à l'homme de l'art suite à la lecture de la description explicative qui fait référence à des exemples qui sont donnés seulement à titre d'illustration et qui ne sauraient en aucune façon limiter la portée de l'invention.

Les exemples font partie intégrante de la présente invention et toute caractéristique apparaissant nouvelle par rapport à un état de la technique antérieure quelconque à partir de la description prise dans son ensemble, incluant les exemples, fait partie intégrante de l'invention dans sa fonction et dans sa généralité.

Ainsi, chaque exemple a une portée générale.

D'autre part, dans les exemples, tous les pourcentages sont donnés en poids, sauf indication contraire, et la température est exprimée en degré Celsius sauf indication contraire, et la pression est la pression atmosphérique, sauf indication contraire.

### EXEMPLES

### Exemple 1 : Couleurs de peau obtenues avant et après bronzage naturel, par phototype

La couleur de la peau de volontaires a été mesurée (en triplicate), avant et après 5 bronzage naturel, par une technique colorimétrique à l'aide d'un Chromamètre CR300 (Minolta, Japon). La chromamétrie permet la quantification objective de variations de couleurs. Les mesures sont données sous la forme de trois paramètres : **L,** a et **b** où **L** mesure la luminosité (du blanc **+L** au noir **-L**), **a** mesure l'aspect "rouge vert" (du rouge **+a** au vert **-a**), et **b** mesure l'aspect "jaune bleu" (du jaune +**b** au bleu -**b**) A partir de ces paramètres, il était possible de calculer l'**A**ngle de **T**ypologie **I**ndividuelle ou I**TA,** défini par la formule simplifiée suivante:
I.T.A. = arc tang [(L*-50)/b*]
L*, a*, et b* signifiant respectivement la moyenne de la valeur de L, a, et de b.

### Phototypes I et II

| Avant bronzage naturel (avant vacances d'été) | | | | |
|---|---|---|---|---|
| | L | a | b | ITA |
| Moyenne n=10 | 62,72 | 9,95 | 18,45 | 0,71 |
| Ecart type | 2,67 | 1,03 | 2,26 | 0,22 |

| Après bronzage naturel (après vacances d'été) | | | | |
|---|---|---|---|---|
| | L | a | b | ITA |
| Moyenne n=10 | 61,07 | 9,57 | 20,08 | 0,57 |
| Ecart type | 3,15 | 2,34 | 2,05 | 0,20 |

### Phototypes III et IV

| Avant bronzage naturel (avant vacances d'été) | | | | |
|---|---|---|---|---|
| | L | a | b | ITA |
| Moyenne n=10 | 56,21 | 11,39 | 20,77 | 0,30 |
| Ecart type | 3,07 | 0,96 | 1,37 | 0,16 |

| Après bronzage naturel (après vacances d'été) | | | | |
|---|---|---|---|---|
| | L | a | b | ITA |
| Moyenne n=10 | 54,54 | 10,90 | 22,25 | 0,22 |
| Ecart type | 4,49 | 1,46 | 1,49 | 0,22 |

On observe que le paramètre a varie peu, alors que L diminue (la peau s'assombrit) et que b augmente (la peau passe du bleu au jaune). Par conséquent, l'indice ITA diminue après exposition au soleil, c'est-à-dire après l'obtention d'un bronzage naturel. Le bronzage naturel induit donc une diminution du paramètre ITA ; la valeur ITA après bronzage est comprise en moyenne entre 70 et 80% de la valeur initiale.

### Exemple 2 :

### a) Préparation d'extraits

### A- Préparation d'extrait végétal de la famille des composées ou astéracées, (extrait de Chicorée) :

1) Introduire en phase aqueuse la plante de chicorée, de préférence la racine de chicorée, après broyage (par exemple introduire dans 95 g d'eau, 5 g de racine à température ambiante pendant 48 heures) ;
2) L'extrait est ensuite filtré ;
3) Le filtrat obtenu est concentré par pulvérisation.
4) Une pâte de chicorée est obtenue.

### B- Préparation d'extrait de la famille des asclépiadacées (Gymnema)

1) Introduire en phase aqueuse la plante de Gymnema, de préférence la feuille de Gymnema, après broyage (par exemple introduire dans 95 g d'eau, 5 g de feuilles à température ambiante pendant 48 heures);
2) L'extrait est ensuite filtré ;
3) Un extrait de Gymnema est obtenu.

### C- Préparation d'extrait de la famille des Oléacées (Muirapuama)

1) Introduire en phase aqueuse la plante de Muirapuama, de préférence la tige et/ou la racine de Muirapuama, après broyage (par exemple introduire dans 95 g d'eau, 5 g de tige et/ou de racine à température ambiante pendant 48 heures) ;
2) L'extrait est ensuite filtré ;
3) Un extrait de Muirapuama est obtenu.

Les extraits des autres végétaux peuvent être obtenus d'une manière similaire.

A partir d'un mélange des 2 extraits, la Chicorée et Muirapuama on obtient en particulier un bronzage très proche du bronzage naturel pour les phototypes I et II dites phototypes pour peaux claires.

À partir d'un mélange des 2 extraits, la Chicorée et Gymnema on obtient en particulier un bronzage très proche du bronzage naturel pour les phototypes III et IV dites phototypes pour peaux mates.

### b) Préparation d'un mélange d'extrait de Chicorée et d'un extrait de la famille des Oléacées (Muirapuama), mise en évidence de l'effet teintant sur peaux claires.

1- Une préparation d'un mélange d'extrait de Chicorée et d'extrait de Muirapuama a été effectuée à raison de 75 % d'extrait de Chicorée pour 25% d'extrait de Muirapuama.
2- La dite préparation a été utilisée à 5% dans une solution hydro-alcoolique (comme par exemple un mélange eau-éthanol).
3- Principe de mesure d'effet teintant par une technique colorimétrique.

Les produits testés ont été appliqués à raison de 5 µl/cm² et la couleur de la peau des volontaires a été mesurée en triplicate par une technique colorimétrique à l'aide d'un Chromamètre CR300 (Minolta, Japon). La chromamétrie permet la quantification objective de variations de couleurs. Les mesures sont données sous la forme des trois paramètres **L**, **a** et **b** comme indiqué précédemment.

Un effet colorant pouvait être justifié si la couleur de la peau était modifiée après l'application du produit testé, se traduisant par une augmentation du paramètre b et une diminution du paramètre L.

L'**A**ngle de **T**ypologie **I**ndividuelle ou **ITA** a été calculé :
Une diminution de ce paramètre **ITA** correspond à un assombrissement de la peau et à une augmentation des pigments mélaniques.

### Phototypes I et II :

| Avant application du mélange | | | | |
|---|---|---|---|---|
| | L | a | b | ITA |
| Moyenne n = 10 | 68,03 | 7,40 | 13,64 | 1,38 |
| Ecart type | 2,14 | 1,29 | 2,28 | 0,39 |

| Après application du mélange | | | | |
|---|---|---|---|---|
| | L | a | b | ITA |
| Moyenne n = 10 | 64,80 | 8,63 | 17,70 | 0,86 |
| Ecart type | 2,39 | 1,13 | 1,96 | 0,22 |

Comme dans le cas d'un bronzage naturel, on observe que le paramètre a varie peu, que le paramètre b augmente (peau plus jaune), et que le paramètre L diminue (peau plus sombre). On remarque ainsi que la valeur de ITA obtenue après application du mélange sur des peaux claires de phototypes I et II est égal à environ 62 % de la valeur initiale. Ceci correspond à une variation de la couleur de la peau comparable à la variation de la couleur de la peau lors d'un bronzage naturel.

### c) Préparation d'un mélange d'extrait de Chicorée et d'un extrait de la famille des Asclépiadacées (Gymnema), mise en évidence de l'effet teintant sur peaux Mates.

1- Une préparation d'un mélange d'extrait de Chicorée et d'extrait de Gymnema a été effectuée à raison de 75 % d'extrait de Chicorée pour 25% d'extrait de Gymnema.
2- La dite préparation a été utilisée à 5% dans une solution hydro-alcoolique (comme par exemple un mélange eau-éthanol).
3- Principe de mesure d'effet teintant par une technique colorimétrique.

### Phototypes III et IV :

| Avant application du mélange | | | | |
|---|---|---|---|---|
| | L | a | b | ITA |
| Moyenne n = 10 | 63,33 | 9,07 | 16,02 | 0,84 |
| Ecart type | 2,86 | 1,38 | 1,67 | 0,23 |

| Après application du mélange | | | | |
|---|---|---|---|---|
| | L | a | b | ITA |
| Moyenne n=10 | 61,18 | 9,53 | 18,68 | 0,60 |
| Ecart type | 2,41 | 0,84 | 1,25 | 0,15 |

Comme dans le cas d'un bronzage naturel, on observe que le paramètre a varie peu, que le paramètre b augmente (peau plus jaune), et que le paramètre L diminue (peau plus sombre). On remarque ainsi que la valeur de ITA obtenue après application du mélange sur des peaux mates de phototypes III et IV est égale à environ 71 % de la valeur initiale. Ceci correspond à une variation de la couleur de la peau comparable à la variation de la couleur de la peau lors d'un bronzage naturel.

### Exemple 3 : Préparation d'un mélange d'extrait de Cichorium intybus L. et de Ptychopetalum Olacoides.

Une préparation d'un mélange d'extrait de Chicorée et d'extrait de Ptychopetalum Olacoides a été effectuée à raison de 75 % d'extrait de Chicorée (voir exemple 2 pour le mode de préparation de l'extrait) pour 25% d'extrait de Ptychopetalum Olacoides (par exemple extrait aqueux réalisé avec 5g de plante, de préférence les tiges et/ou racines, et 95g d'eau, à température ambiante pendant 48 heures).

La dite préparation a été utilisée à 5% dans une solution hydro-alcoolique. Les résultats obtenus sont similaires à ceux obtenus à l'exemple 2b.

### Exemple 4 : Préparation d'un mélange d'extrait de Cichorium intybus L. et de Gymnema Sylvestre.

Une préparation d'un mélange d'extrait de Chicorée et d'extrait de Gymnema Sylvestre a été effectuée à raison de 75 % d'extrait de Chicorée (voir exemple 1 pour le mode de préparation de l'extrait) pour 25% d'extrait de Gymnema Sylvestre (par exemple extrait aqueux réalisé avec 5g de plante, de préférence les feuilles, et 95g d'eau, à température ambiante pendant 48 heures). La dite préparation a été utilisée à 5% dans une solution hydro-alcoolique. Les résultats obtenus sont similaires à ceux obtenus à l'exemple 2c.

### Exemple 5 : Préparation d'un mélange d'extrait de Cichorium intybus L. et d'un extrait de la famille des Composées (ou astéracées) et en Particulier de Cynara Scolymus.

Au cours de l'étape 1 de l'exemple 3 et/ou 4, un extrait de Cynara Scolymus est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2c.

### Exemple 6 : Préparation d'un mélange d'extrait de Cichorium intybus L. et d'un extrait de la famille des Labiacées et en particulier de Mentha Piperta.

Au cours de l'étape 1 de l'exemple 3 et/ou 4, un extrait de Mentha Piperta est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2b.

### Exemple 7 : Prépraration d'un mélange d'extrait de Cichorium intybus L. et d'un extrait de la famille des Malvacées et en particulier de Malva Silvestris.

Au cours de l'étape 1 de l'exemple 3 et/ou 4, un extrait de Malva Silvestris est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2c.

### Exemple 8 : Préparation d'un mélange d'extrait de Cichorium intybus L. et d'un extrait de la famille des Ternstroemiacées et en particulier de Thea Sinensis.

Au cours de l'étape 1 de l'exemple 3 et/ou 4, un extrait de Thea Sinensis est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2c.

### Exemple 9 : Préparation d'un mélange d'extrait de Cichorium intybus L. et d'un extrait de la famille des Juglandacées et en Particulier de Juglans Regia.

Au cours de l'étape 1 de l'exemple 3 et/ou 4, un extrait de Juglans Regia est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2b.

### Exemple 10 : Préparation d'un mélange d'extrait de Cichorium intybus L. et d'un extrait de la famille des Lythracées et en particulier de Lawsonia Inermis.

Au cours de l'étape 1 de l'exemple 3 et/ou 4, un extrait de Lawsonia Inermis est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2b.

### Exemple 11 : Préparation d'un mélange d'extrait de Cichorium intybus L. et d'un extrait de la famille des Fagacées et en particulier de Castanea Vulgaris.

Au cours de l'étape 1 de l'exemple 3 et ou 4, un extrait de Castanea Vulgaris est préparé (par exemple extrait aqueux réalisé avec 5g de plante, de préférence les feuilles, et 95g d'eau, à température ambiante pendant 48 heures).

Toutes les autres étapes sont identiques.

Les résultats obtenus sont similaires à ceux obtenus à l'exemple 2c.

### Exemple 12 : Autres plantes sélectionnées pour leurs capacités à colorer la peau.

L'ensemble des plantes suivantes a été sélectionné sur la base des critères de la présente invention, et en particulier sur l'effet technique que ces plantes apportent en reproduisant le bronzage naturel de la peau. Dans chaque cas, les extraits aqueux correspondants ont été réalisés selon le procédé décrit préalablement (par exemple 5g de plante, 95g d'eau, extraction à température ambiante pendant 48 heures, filtration et utilisation de l'extrait).
- Menthe piperta (de préférence feuilles),
- Malva silvestris (de préférence feuilles),
- Cynara scolymus (de préférence feuilles),
- Thea sinensis (de préférence feuilles),
- Juglans regia (de préférence feuilles),
- Lawsonia inermis (de préférence feuilles),
- Castanea vulgaris (de préférence feuilles),
- Asarum europaeum (de préférence feuilles),
- Leonurus cardiaca (de préférence fleurs),
- Ballota foetida (de préférence fleurs),
- Ocimum basilicum (de préférence fleurs et/ou feuilles),
- Stachys officinalis (de préférence racines et/ou feuilles),
- Brunella vulgaris (de préférence fleurs et/ou feuilles et/ou tiges),
- Calamintha officinalis (de préférence feuilles et/ou tiges),
- Thymus vulgaris (de préférence feuilles),
- Rosmarinus officinalis (de préférence feuilles),
- Humulus lupulus (de préférence cônes),
- Vaccinium myrtillus (de préférence baies),
- Arctotaphylos uva-ursi (de préférence feuilles),
- Calluna vulgaris (de préférence fleurs),
- Artemisia abisinthium (de préférence fleurs),
- Artemisia vulgaris (de préférence racines et/ou feuilles),
- Artemisia abrotonum (de préférence feuilles et/ou tiges),
- Artemisia glacialis (de préférence racines et/ou fleurs),
- Artemisia mutellina (de préférence racines et/ou fleurs),
- Artemisia spicata (de préférence racines et/ou fleurs),
- Chamaemelum nobile (de préférence tiges et/ou fleurs),
- Fraxinus excelsior (de préférence écorce),
- Syringa vulgaris (de préférence feuille),
- Jasminium grandiflorum (de préférence fleur),
- Lythrum salicaria (de préférence tiges et/ou fleurs et/ou feuille),
- Althaea officinalis (de préférence racines et/ou feuilles),
- Hysopus officinalis (de préférence fleurs),
- Origanum majorana (de préférence feuille),
- Salvia officinalis (de préférence feuille),
- Melissa officinalis (de préférence feuille),
- Melittis melissophyllum (de préférence tiges et/ou fleurs et/ou feuille),
- Lavandula officinalis (de préférence fleurs),
- Quercus robur (de préférence écorce),
- Fagus silvatica (de préférence écorce),
- Nepta cataria (de préférence tiges et/ou fleurs et/ou feuille),
- Origanum dictamus (de préférence tiges et/ou fleurs et/ou feuille),
- Thymus serpyllum (de préférence fleurs),

### Exemple 13: Utilisation d'un mélange d'un extrait de chicorée (Cichorium Intybus) ou d'une plante de la famille des Composées, combiné.

- Soit avec un extrait de Gymnema (Gymnema Sylvestre) ou un extrait de la famille des Asclepiadacées,
- Soit avec un extrait de Muirapuama (Ptychopetalum Olacoides) ou un extrait de la famille des Oléacées.
De cette manière il est possible de réaliser des compositions comprenant 75 % d'un extrait de chicorée (Cichorium Intybus) ou d'une plante de la famille des Composées, et 25 % d'un extrait de la famille des Asclepiadacées comme par exemple Gymnema (Gymnema Sylvestre), ou de la famille des Oléacées comme par exemple Muirapuama (Ptychopetalum Olacoides).

La composition obtenue est tout à fait efficace pour obtenir des modifications de la couleur de la peau semblables à celles obtenues lors d'un bronzage naturel.

### Exemple 14 de l'invention : Exemple de formulation contenant les Produits de l'invention.

Utilisation des produits de l'invention dans des formulations cosmétiques ou pharmaceutiques de type émulsion huile dans eau

| Formulation 14a : | | |
|---|---|---|
| | Eau | qsp 100 |
| | Butylene Glycol | 2 |
| | Glycerine | 3 |
| | Sodium Dihydroxycetyl | 2 |
| Phosphate, | | |
| | Isopropyl Hydroxycetyl | |
| Ether | | |
| | Glycol Stearate SE | 14 |
| | Triisononaoin | 5 |
| | Octyl Cocoate | 6 |
| | Butylene Glycol, | 2 |
| Methylparaben, | | |
| | Ethylparaben, | |
| Propylparaben, | | |
| | pH ajusté à 5,5 | |
| | Produits de l'invention | 0,01 - 20 % |

| Formulation 14b : | | |
|---|---|---|
| | Eau | qsp 100 |
| | Butylene Glycol | 2 |
| | Glycerine | 3 |
| | Polyacrylamide, | 2,8 |
| Isoparafin, Laureth-7 | | |
| | Butylene Glycol, | 2 |
| Methylparaben, | | |
| | Ethylparaben, | |
| Propylparaben ; | | 2 |
| | Phenoxyethanol, | |
| Methylparaben, | | |
| | Propylparaben, | 0,5 |
| Butylparaben, Ethylparaben | | |
| | Butylene Glycol | |
| | Produits de l'invention | 0,01- 20 % |

| Formulation 14c : | | |
|---|---|---|
| | Carbomer | 0,50 |
| | Propylene Glycol | 3 |
| | Glycerol | 5 |
| | Eau | qsp 100 |
| | Octyl Cocoate | 5 |
| | Bisabolol | 0,30 |
| | Dimethicone | 0,30 |
| | Sodium Hydroxide | 1,60 |
| | Phenoxyethanol, | 0,50 |
| Methylparaben, | | |
| | Propylparaben, | |
| Butylparaben, Ethylparaben | | |
| | Parfum | 0,30 |
| | Produits de l'invention | 0,01- 20 % |

### Exemple 15 de l'invention : Utilisation des produits de l'invention dans une formulation de type eau dans huile.

| | | |
|---|---|---|
| | PEG 30 - | 3 |
| dipolyhydroxystearate | | |
| | Capric Triglycérides | 3 |
| | Cetearyl Octanoate | 4 |
| | Dibutyl Adipate | 3 |
| | Grape Seed Oil | 1,5 |
| | Jojoba Oil | 1,5 |
| | Phenoxyethanol, | 0,5 |
| Methylparaben, | | |
| Propylparaben, Butylparaben, | | |
| Ethylparaben | | |
| | Glycerine | 3 |
| | Butylene Glycol | 3 |
| | Magnesium Sulfate | 0,5 |
| | EDTA | 0,05 |
| | Eau | qsp 100 |
| | Cyclomethicone | 1 |
| | Dimethicone | 1 |
| | Parfum | 0,3 |
| | Produits de l'invention | 0,01 - 20 % |

### Exemple 16 de l'invention : Utilisation des produits de l'invention dans une formulation de type shampoing ou gel douche.

| | | | |
|---|---|---|---|
| | Xantham Gum | | 0.8 |
| | Eau | | qsp 100 |
| | Butylene | Glycol, | 0,5 |
| Methylparaben, | | | |
| | Ethylparaben, | | |
| Propylparaben | | | |
| | Phenoxyethanol, | | 0,5 |
| Methylparaben, | | | |
| | Propylparaben, | | |
| Butylparaben, Ethylparaben | | | |
| | Citric acid | | 0,8 |
| | Sodium Laureth Sulfate | | 40,0 |
| | Produit de l'invention | | 0,01 - 20% |

### Exemple 17 de l'invention : Utilisation des produits de l'invention dans une formulation de gels aqueux (contours de l'oeil, amincissants, etc.).

| | | |
|---|---|---|
| | Eau | qsp 100 |
| | Carbomer | 0,5 |
| | Butylene Glycol | 15 |
| | Phenoxyethanol, | 0,5 |
| Methylparaben, Propylparaben, | | |
| Butylparaben, Ethylparaben | | |
| | Produits de l'invention | 0,01- 20% |

### Exemple 18 : Etudes toxicologiques des produits de l'invention.

### Evaluation de l'acceptation cosmétique d'une préparation contenant le sujet de l'invention

Les essais de toxicologie ont été réalisés sur le composé obtenu selon l'exemple 2 ou 3 incorporé à 10% dans un gel de xanthane à 0,5%, par une évaluation oculaire chez le lapin, par l'étude de l'absence de toxicité anormale par administration orale unique chez le rat et par l'étude du pouvoir sensibilisant sur le cobaye.

### Evaluation de l'irritation primaire cutanée chez le lapin :

Les préparations décrites ci-dessus sont appliquées sans dilution à la dose de 0,5 ml sur la peau de 3 lapins selon la méthode préconisée par la directive OCDE concernant l'étude de « l'effet irritant/corrosif aigu sur la peau ».

Les produits sont classés selon les critères définis par l'arrêté du 1/2/1982 publié au JORF du 21/02/82.

### Evaluation de l'irritation oculaire chez le lapin :

Les préparations décrites ci-dessus ont été instillées pure en une seule fois, à raison de 0,1ml, dans l'oeil de 3 lapins selon la méthode préconisée par la directive de l'OCDE n°405 du 24 février 1987 concernant l'étude de « l'effet irritant/corrosif aigu sur les yeux ».

Les résultats de ce test permettent de conclure que les préparations peuvent être considérées comme non irritantes pour les yeux, au sens de la directive 91/326 CEE utilisée pure ou sans dilution.

### Essai sur l'absence de toxicité anormale par administration orale unique chez le rat :

Les préparations décrites ont été administrées en une fois par voie orale à la dose de 5g/Kg de poids corporel, à 5 rats mâles et 5 rats femelles selon un protocole inspiré de la directive de l'OCDE n°401 du 24 février 1987 et adapté aux produits cosmétiques,

Les DL0 et DL50 sont trouvées supérieures à 5000 mg/Kg. Les préparations testées ne sont donc pas classées parmi les préparations dangereuses par ingestion.

### Evaluation du potentiel de sensibilisation cutanée chez le cobaye :

Les préparations décrites sont soumises au test de maximisation décrit par Magnusson et Kligmann, protocole en accord avec la ligne directrice n°406 de l'OCDE.

Les préparations sont classées comme non sensibilisantes par contact avec la peau.

## Revendications

1. Utilisation d'un extrait végétal de la famille des composées ou astéracées, de préférence un extrait de chicorée sauvage (Cichorium intybus L.), pour teinter un tissu cutané d'un sujet, et en particulier pour colorer ce tissu, et de préférence pour augmenter l'aspect bronzé ou l'aspect doré (couleur jaune) de ce tissu cutané.

2. Utilisation d'un extrait végétal de la famille des composées ou astéracées, de préférence un extrait de chicorée sauvage pour teinter, en particulier pour colorer, un tissu cutané de phototype I et/ou II d'un sujet, de préférence pour augmenter l'aspect bronzé ou l'aspect doré (couleur jaune) de ce tissu.

3. Utilisation selon la revendication 2, **caractérisée en ce que** l'extrait de végétal de la famille des composées ou astéracées, de préférence un extrait chicorée sauvage, est en combinaison avec un extrait de la famille des Oléacées, de préférence un extrait de Muirapuama (Ptychoetalum Olacoides).

4. Utilisation d'un extrait de chicorée sauvage pour teinter, en particulier pour colorer, un tissu cutané de phototype III et/ou IV d'un sujet, de préférence pour augmenter l'aspect bronzé ou l'aspect doré (couleur jaune) de ce tissu.

5. Utilisation selon la revendication 4, **caractérisée en ce que** l'extrait de chicorée sauvage est en combinaison avec un extrait d'un végétal de la famille des asclépiadacées, de préférence un extrait de Gymnema Sylvestre.

6. Utilisation d'un extrait végétal de la famille des composées ou astéracées, de préférence un extrait de chicorée sauvage, en combinaison avec un extrait végétal choisi parmi le groupe consistant en :
Menthe piperta ; Malva silvestris ; Cynara scolymus ;Thea sinensis ;Juglans regia ; Lawsonia inermis; Castanea vulgaris; Asarum euroaeum ; Leonurus cardiaca; Ballota foetida; Ocimum basilicum; Stachys officinalis ; Brunella vulgaris ; Calamintha officinalis ; Thymus vulgaris ; Rosmarinus officinalis ; Humulus lupulus ; Vaccinium myrtillus ; Arctotaphylos uva-ursi ; Calluna vulgaris ; Artemisia abisinthium ; Artemisia vulgaris, Artemisia abrotonum ; Artemisia glacialis ; Artemisia mutellina ; Artemisia spicata ; Chamaemelum nobile ; Fraxinus excelsior ; Syringa vulgaris ; Jasminium grandiflorum ; Lythrum salicaria ; Althaea officinalis ; Hysopus officinalis ; Origanum majorana ; Salvia officinalis ; Melissa officinalis ; Melittis melissophyllum ; Lavandula officinalis ,Quercus robur ; Fagus silvatica ; Nepta cataria ; Origanum dictamus ; Thymus serpyllum, pour reproduire un bronzage naturel sur au moins une partie du tissu cutané d'un sujet.

7. Procédé de préparation d'une composition, notamment d'une composition cosmétique, comprenant le mélange d'un extrait végétal de la famille des composées ou astéracées, de préférence un extrait de chicorée sauvage, avec un extrait d'un végétal choisi parmi le groupe consistant en :
Menthe piperta ; Malva silvestris ; Cynara scolymus ;Thea sinensis ;Juglans regia ; Lawsonia inermis ; Castanea vulgaris ; Asarum euroaeum ; Leonurus cardiaca; Ballota foetida ; Ocimum basilicum ; Stachys officinalis ; Brunella vulgaris ; Calamintha officinalis; Thymus vulgaris ; Rosmarinus officinalis ; Humulus lupulus ; Vaccinium myrtillus ; Arctotaphylos uva-ursi ; Calluna vulgaris ; Artemisia abisinthium ; Artemisia vulgaris, Artemisia abrotonum ; Artemisia glacialis ; Artemisia mutellina ; Artemisia spicata ; Chamaemelum nobile ; Fraxinus excelsior ; Syringa vulgaris ; Jasminium grandiflorum ; Lythrum salicaria ; Althaea officinalis ; Hysopus officinalis ; Origanum majorana ; Salvia officinalis ; Melissa officinalis ; Melittis melissophyllum ; Lavandula ofhcinalis ,Quercus robur ; Fagus silvatica ; Nepta cataria ; Origanum dictamus ; Thymus serpyllum.

8. Composition cosmétique comprenant un extrait végétal de la famille des composées ou astéracées, de préférence un extrait de chicorée sauvage et un extrait d'un végétal choisi parmi le groupe consistant en :
Menthe piperta ; Malva silvestris ; Cynara scolymus ;Thea sinensis ;Juglans regia ; Lawsonia inermis ; Castanea vulgaris ; Asarum europaeum ; Leonurus cardiaca; Ballota foetida ; Ocimum basilicum ; Stachys officinalis ; Brunella vulgaris ; Calamintha officinalis ; Thymus vulgaris ; Rosmarinus officinalis ; Humulus lupulus ; Vaccinium myrtillus ; Arctotaphylos uva-ursi ; Calluna vulgaris ; Artemisia abisinthium ; Artemisia vulgaris, Artemisia abrotonum ; Artemisia glacialis ; Artemisia mutellina ; Artemisia spicata ; Chamaemelum nobile ; Fraxinus excelsior ; Syringa vulgaris ; Jasminium grandiflorum ; Lythrum salicaria ; Althaea officinalis ; Hysopus officinalis ; Origanum majorana ; Salvia officinalis ; Melissa officinalis ; Melittis melissophyllum ; Lavandula officinalis ,Quercus robur ; Fagus silvatica ; Nepta cataria ; Origanum dictamus ; Thymus serpyllum.

9. Procédé de criblage d'un extrait végétal pour la fabrication d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage;
b) l'application d'un mélange d'un extrait végétal ou d'un mélange d'extraits végétaux sur au moins une partie d'un tissu cutané ;
c) la sélection de l'extrait végétal ou du mélange d'extraits végétaux lorsque la variation de l'intensité de couleur du tissu cutané avant et après application à l'étape b) reproduit la variation de l'intensité de couleur obtenue à l'étape a).

10. Procédé de criblage d'un extrait végétal pour la fabrication d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage;
b) l'application d'un mélange d'un extrait de la famille des composées ou astéracées, de préférence un extrait de chicorée sauvage, et d'un extrait végétal sur au moins une partie d'un tissu cutané ;
c) la sélection de l'extrait végétal lorsque la variation de l'intensité de couleur du tissu cutané avant et après application à l'étape b) reproduit la variation de l'intensité de couleur obtenue à l'étape a).

11. Procédé de préparation d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage;
b) l'application d'un mélange d'un extrait végétal ou d'un mélange d'extraits végétaux sur au moins une partie d'un tissu cutané ;
c) la sélection de l'extrait végétal ou du mélange d'extraits végétaux lorsque la variation de l'intensité de couleur du tissu cutané avant et après application à l'étape b) reproduit la variation de l'intensité de couleur obtenue à l'étape a).
d) la formulation du mélange de l'extrait végétal ou du mélange d'extraits végétaux, pour obtenir une composition cosmétique pour teinter, en particulier pour colorer au moins une partie d'un tissu cutané d'un sujet.

12. Procédé de préparation d'une composition cosmétique pour teinter, en particulier pour colorer, au moins une partie d'un tissu cutané d'un sujet comprenant :
a) la détermination de la variation de l'intensité de couleur d'un tissu cutané avant et après bronzage;
b) l'application d'un mélange d'un extrait de la famille des composées ou astéracées, de préférence un extrait de chicorée sauvage et d'un extrait végétal sur au moins une partie d'un tissu cutané ;
c) la sélection du végétal lorsque la variation de l'intensité de couleur du tissu cutané avant et après application à l'étape b) reproduit la variation de l'intensité de couleur obtenue à l'étape a).
d) la formulation du mélange de l'extrait végétal et de l'extrait végétal de la famille des composées ou astéracées, de préférence un extrait de la chicorée sauvage, pour obtenir une composition cosmétique pour teinter, en particulier pour colorer au moins une partie d'un tissu cutané d'un sujet.
